# EUROPEAN PATENT APPLICATION

(11) **EP 1 739 421 A1**
(43) Date of publication of application: **03.01.2007**
(21) Application number: 05425459.4
(22) Date of filing: 27.06.2005
(51) Int. Cl.: G01N 33/18, G01N 27/38, G01N 27/49

(54) **Electrochemical analyser for the selective measurement of chlorites in water**

(71) Applicant: CLR Srl, 20090 Rodano Millepini MI (IT)
(72) Inventor: Castioni, Luigi, 20132 Milan (IT)

(57) **Abstract**

An online electrochemical analyser for the measurement of chlorites in water characterized by high selectivity even in the presence of interfering substances normally present with chlorites in drinking water plants and in all those plants where chlorine dioxide or chlorites are used as disinfectants and/or as oxidising agents.
Selectivity of the measurement is obtained by the optimization of parameters:
. electrode construction (e.g. material) and electrode dimensioning;
. the position of each electrode with respect to the other electrodes;
. hydraulic arrangement in the electrode holder (cell);
. the depolarizing system;
. the polarization voltage;
Advantages:
. selectivity for chlorites even in presence of the interfering substances normally included in the water to be monitored.
. long time reliability of the measure thanks to the depolarizing system and to the stability of the chosen reference electrode.
. low maintenance requirements because there is no moving part subject to wear, no physical cleaning means (e.g. sand) to be replaced, no mechanical cleaning system (e.g. as brushes) subject to wear.
. very low operating cost, since no reagent addition is required for the measurement.

## Description

### Technical field of the invention

Chlorite ion is one of the reaction by products appearing with the use of chlorine dioxide, a substance typically used for the oxidation and disinfection of drinking water, wastewater and industrial water.
The Council Directive 98/83/EC of 3 November 1998 concerning the quality of water intended for human consumption and following actuation in different European Countries (e.g. D.Lgs.31/2001 for Italy) give a limit for the concentration of chlorites in such waters (200 µg/L as chlorites).
This means that drinking water plants applying chlorine dioxide in one or more stages of their treatment should monitor chlorites concentration at least in the water at the outlet of the plant (water to distribution system).
Chlorites in water are always or almost always contemporarily present in the same water with chlorine dioxide: for this reason the analyser for chlorites must be selective to chlorites and insensitive to chlorine dioxide.
In waters intended for human consumption that have undergone different treatment processes (e.g. chlorination, ozone addition, bromine addition, treatment with permanganate, peracetic acid, hydrogen peroxide etc.) chlorites will always be associated with the other substances added to the water: the analyser must therefore be insensitive to the interference of all the substances potentially present in the water together with chlorites.

### Background art

The invention subject of this patent concerns an on-line electrochemical analyser for the measure of chlorites in water characterized by high selectivity even in presence of interfering substances normally present with chlorites in drinking water plants and in all those plants where chlorine dioxide or chlorites are used as disinfectants and/or as oxidizing agents.

Other chlorite analysers have been developed and are actually present in the market, but present invention introduces an innovative feature: the real selectivity of the measure, obtained by the optimization of many important parameters:
- electrodes construction material and electrodes dimensioning;
- the position of each electrode in respect to the other electrodes;
- hydraulic arrangement in the electrode holder (cell);
- the depolarizing system;
- the polarization voltage.

### Brief description of the drawings

Fig.1 is the schematic representation of the on line electrochemical analyser for selective measurements of chlorites in water using the cell suitable to work with higher flowrates
   In this drawing:
   - Fig.1 (A): represents the sample inlet to the measuring cell
   - Fig.1 (B): represents the sample overflow drain used only when the cell is installed in the through-flow arrangement
   - Fig.1 (C): represents the sample outlet that is active both in the through flow installation and in the in-line installation arrangement
   - Fig.1 (D): represents the flow switch which senses the absence of sample flow into the cell and, in case, provides a contact closure
   - Fig.1 (E): represents the temperature sensor
   - Fig.1 (F): represents the counter electrode (C)
   - Fig.1 (G): represents the combined measuring/reference electrodes [working electrode (W) and reference electrode (R) are combined into a single rod]
   - Fig.1 (H): represents the solution ground pin
   - Fig.1 (I): represents the system capable to keep constant sample velocity and sample flow rate inside the electrodes chamber [Fig.1(M)]
   - Fig.1 (L): represents the electronic unit
   - Fig.1 (M): represents the electrode chamber
   - Fig.1 (N): represent the shielded cables for the connection sensor to electronic unit
Fig.2 is the schematic representation of the on line electrochemical analyser for selective measurement of chlorites in water using the cell suitable to work with lower flowrates
   In this drawing:
   - Fig.2 (A): represents the sample inlet
   - Fig.2 (B): represents the sample outlet (drain)
   - Fig.2 (C): represents the counter electrode (C)
   - Fig.2 (D): represents the combined measuring/reference electrodes [working electrode (W) and reference electrode (R) are combined into a single rod]
   - Fig.2 (E): represents the electronic unit
   - Fig.2 (F): represents the electrode chamber
   - Fig.2 (G): represent the shielded cables for the connection sensor to electronic unit

### Disclosure of the invention

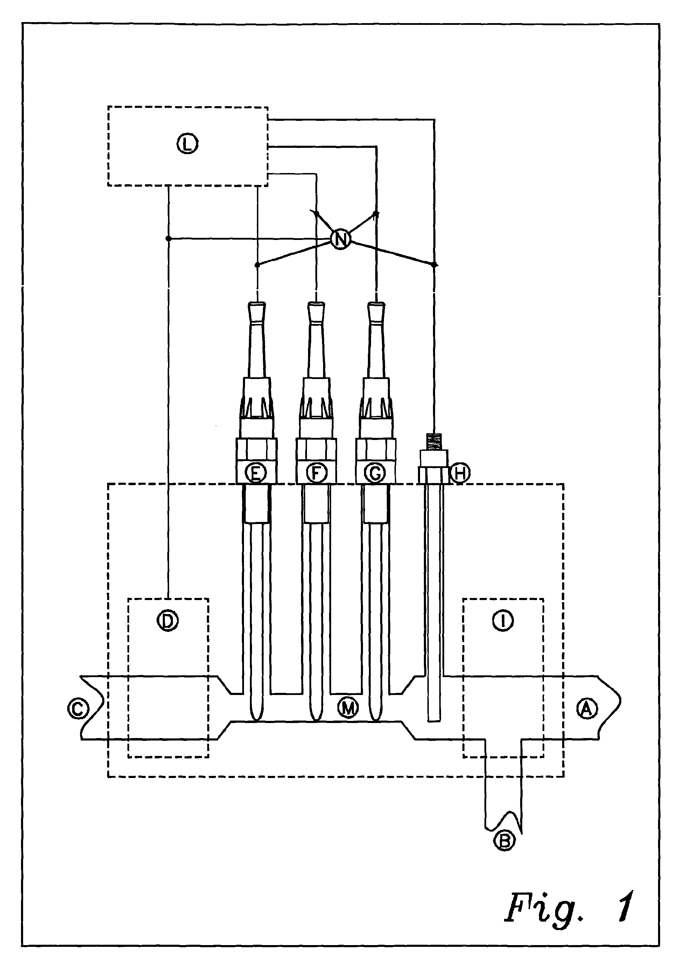

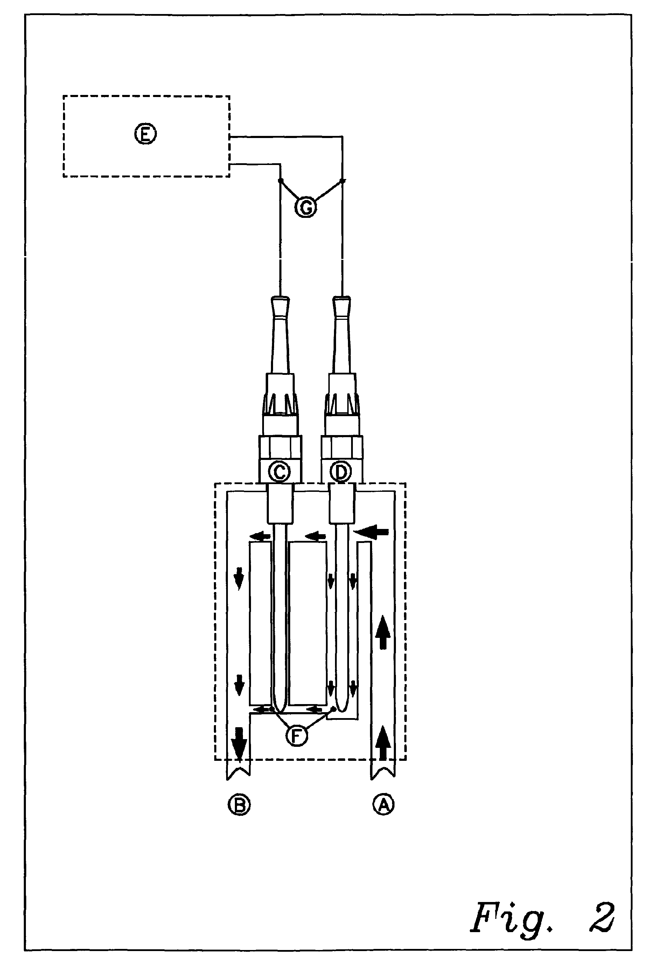

The on-line electrochemical analyser for selective measurements of chlorites object of this patent is composed of:
- an electrode holder (also called cell), that may be built with different arrangements to suit many different application needs (process pressure, sample flow rate, type on installation). Refer to Fig.1 and Fig.2 to have some examples of possible cell configuration.
- three electrodes for the measure of chlorites [Fig.1(F) and Fig.1(G); Fig.2(C) and Fig.2(D)] and a temperature sensor [Fig.1(E)]
- shielded cables c/w connectors on electrode side \for the connection to electronic unit [Fig.1(N) and Fig.2 (G)]
- an electronic unit receiving the signal from the sensor and processing it in order to obtain the local indication of chlorites concentration and a proportional analogue signal for remote retransmission [Fig.1 (L) and Fig.2(E)].

Measuring electrodes are three: working electrode (W), counter electrode (C), and reference electrode, (R), and they are located into the cell as indicated in Fig.1 and in Fig.2.
Operating principle for this 3 electrodes potentiostatic measuring system is the following:
the electronic circuit impresses to the working electrode a polarization voltage so that the potential of this electrode (W) corresponds to the discharge voltage of chlorites in the specific operating conditions, then the electronic circuit acts in order to keep constant the potential of working electrode in respect to the potential of the reference electrode (R). The potential given by the reference electrode is very stable along time.
When a deviation between the nominal potential of the working electrode (W) and the reference potential occurs the electronic circuit corrects this condition by circulating a current between the counter electrode (C) and the measuring electrode (W).
The a.m. deviation may only occur if some substance reacts to the measuring electrode subtracting electrons.
The construction material for the electrodes, the electrodes dimensioning, the surface treatment on the electrodes themselves and the chosen polarization voltage have all been selected so that only chlorite ions can react (subtract electrons) to the working electrode.
So, when chlorite ions are present in the sample, they discharge on the measuring electrode (W) subtracting electrons; the circuit will work to re-establish the potential and a current will circulate across the counter electrode and the working electrode: This current exactly corresponds to the discharge current generated by the reacting chlorites (that is, to the number of electrons subtracted by chlorite ions) which is proportional to the concentration of chlorites in the sample.
The electronic unit measures the current flowing between the C and W electrodes and processes it to obtain the chlorite concentration in the sample as ppm ClO₂⁻.

One of the advantages of this invention is that no reagent addition is required to operate the measurement.
Analyzers which require a sample conditioning system (addition of reagent) are characterized by high operating costs due to reagent consumption (cost of the chemical) and to time waste in preparing the solution and refilling the reservoir.

The working electrode (W) and the counter electrode (C) are made of graphite with a special surface treatment designed for minimizing the naturally occurring polarization of the electrodes during they normal operation (this is a common phenomenon that occurs on all measuring electrodes and on all counter electrodes and is intrinsic to the operation of the electrodes themselves).
Another function of the surface treatment for the C and W electrodes is to reduce ground noises.
Shape and dimensions of the W and C electrodes have been designed for optimizing current density on the reactive surfaces: the current density is high enough for the discharge reaction of chlorite ions, but not so high to allow polarization of the electrodes due to deposit of polarizing film (polarization phenomenon is already minimized by the surface treatment as described here above).

The reference electrode is a classic Ag/AgCl reference with KCI electrolyte saturated with AgCl, and in typical operating conditions assures a long term stability of the reference voltage.
The reference electrode may also be made of calomel (Hg/Hg₂Cl₂), with saturated KCI electrolyte, for those applications where a very high measurement stability is required. The reference electrode can also be of the following types: Hg/HgSO₄, Hg/HgO, Hg/ThCl in order to well suit many different application needs.

The three electrodes, W, C and R and the temperature sensor are installed into the electrode holder (cell).

The main duty of the cell is to keep constant the sample flow rate and the sample velocity inside the electrodes chamber [Fig.1(M) and Fig.2 (F)], regardless the sample flow rate and pressure at the inlet of the cell itself.

As previously stated electrodes (and mainly working electrodes and counter electrodes), even if chemically inert, become polarized during the measure because of concentration/diffusion phenomena of reaction products and chlorites. This behaviour is intrinsic to the electrochemical process according to which the measure is carried out and causes a gradual loss of sensitivity along time.
A depolarization system (either mechanical, phisical or electronic) must therefore be foreseen for all electrodes of this kind, otherwise the measure will decrease down to zero in a more or less short time.
The analyser subject of the present patent includes an innovative depolarization system: depolarization is operated just by the sample flowing into the electrodes chamber.
The hydraulic arrangement [Fig.1(I) and Fig.2 (A+B+F)] of the cell (in all available versions) has been designed so that:
1. a high turbulence is created on the sample flowing inside the electrodes chamber; this condition of the flow rapidly removes the polarizing film on the electrodes surfaces; the electrodes surfaces are therefore kept always active.
2. the sample velocity inside the electrodes chamber is kept constant regardless the sample flow rate variations at the inlet of the cell.
Allowed conditions are, for the cell as schematically represented in Fig.2:
sample flow rate 15 l/h to 200 l/h, through flow cell installation, atmospheric operating pressure.
Allowed conditions are, for the cell as schematically represented in Fig.1:
- Through-flow arrangement: sample flow rate 60 l/h to 1000 l/h, atmospheric operating pressure
- Direct in-line installation arrangement: sample flow rate 60 l/h to 1000 l/h, operating pressure up to 4 bar.
The velocity of the portion of sample flowing into the electrodes chamber [Fig.1(M) and Fig.2 (F)], is so high that the film causing electrodes passivation cannot form (deposit) on the electrode surfaces.
The advantage of not having mechanical depolarizing systems (brushes, abrasive surfaces etc.) nor physical depolarizing systems (balls, sand etc.) nor moving parts concerns the dramatic decrease in maintenance requirements (all the a.m. means are subject to wear and require periodical maintenance and replacement).
A further advantage of the hydraulic arrangement designed for the cell included in the subject of this patent is that the sample velocity prevents deposits of debris that could block the passages inside the cell: monitoring interruption for cell cleaning is therefore very seldom required.
The cell also includes a flow switch [Fig.1(D)] that gives a contact closure in case the sample flow rate becomes zero: the electronic units receives the contact closure, gives a local indication of the alarm and retransmits the alarm condition to prevent wrong chlorite readings in absence of flow.
A solution ground pin [Fig.1(H)], prevents negative effects of stray currents on the measure.

Cables for the connection of each electrode and of the temperature sensor to the electronic unit are coaxial shielded type, so minimizing electro-magnetic interferences on the signal transmission; this allows to position the cell at variable distances form the electronic unit, from few cm up to several meters, without interferences nor ground noise on the signal. [Fig.1(N) and Fig.2 (G)].

The electronic unit (transmitter) [Fig.1(L) and Fig.2 (E)] basically includes the following functionalities:
- it generates the polarization voltage impressed to the electrodes
- it receives the current signal proportional to chlorite ion concentration in the sample
- it amplifies the a.m. signal as required
- it computes temperature compensation of the measure using a dedicated algorithm (temperature of the sample is measured by the temperature sensor, which signal enters the electronic unit)
- it then corrects the temperature compensated signal on the basis of the values introduced with the calibration of the system
- it generates the value of chlorites to be displayed
- it generates the proportional analogue output, galvanically isolated.
Let's see with some more detail the a.m. transmitter functionalities:
Polarization voltage impressed by the electronic unit to the measuring electrode has been chosen so that it is the chlorite ion discharge voltage on the working electrode in the system operating conditions (mentioned above).
Allowed limits for the polarization voltage are narrow in order to avoid the discharge of other substances, i.e. the chlorine dioxide, which discharge voltage is quite near the discharge voltage of chlorites, but the same is for all the other possible interfering substances, including oxygen, that is always present in the sample.
Possible interfering substances in the chlorite ion measurements may be present for many reasons:
   - some of them are residuals or by products of processes used to treat the water , among them: chlorine dioxide, ClO₂, chlorine, Cl₂, ipochlorites, ClO⁻, bromine, Br₂, bromates, BrO₃⁻, potassium permanganate, KMnO₄, ozone, O₃, peracetic acid CH₃CO₃H, hydrogen peroxide, H₂O₂, ferrous chloride FeCl₂, ferric chloride FeCl₃
   - some other are normally present in the water to be treated, among them: oxygen O₂, iron Fe(II) and Fe(III), Manganese and other.
The circuit generating the polarization voltage and measuring the input signal from the cell can keep constant the value of generated polarization voltage (so forth assure an high measurement accuracy) regardless the variations of some parameters that normally occur from one application to one other and even within the same application from time to time, as , for example:
- variations in chlorites concentration
- variations of the pH value
- variations of the Oxidation Reduction Potential (ORP)
- variation in dissolved salts concentration (conductivity)
- variable presence of calcium, iron, manganese ions and of ferrous chloride.
The input signal to the cell (chlorites discharge current) is amplified and compensated for the influence of temperature variations.
Temperature compensation is operated according to actual sample temperature, measured by the temperature sensor inside the cell. a dedicated algorithm, specifically designed for chlorites measurements is used; the same algorithm allows a good flexibility so that it can be adapted to any application need just modifying some configuration parameters in the transmitter.
The signal, once thermo compensated, is corrected according to calibration data, then displayed as ppm ClO₂⁻ and retransmitted on the analogue output 4÷20 mA or 0÷10 V, galvanically isolated.
The electronic unit can also drive the cleaning of the sensor, operated with water or water and specific detergent, with configurable frequency and duration.

### Advantages and innovation given by the invention

Most innovative features of the invention subject of the present patent may be resumed as follows:
1. Selectivity for chlorites even in presence of interfering substances normally associated to chlorites in the water to be measured.
2. Long term reliability of the measuring system due to the chosen depolarizing system and to the good stability of reference electrode.
3. Very low maintenance requirements since there is no moving part subject to wear, nor physical cleaning means (as sand) to be replaced, nor mechanical cleaning system (as bushes) again subject to wear
4. Very low operating cost, since no reagent addition is required for the measure.
5. Wide measuring range, 0÷10.00 ppm, with very good resolution (0,01 ppm)

### Industrial applicability of the invention

Typical application of the on-line electrochemical analyser for selective chlorite measurements is in drinking water plants, to monitor chlorites concentrations in the various stages of the treatment:
- wherever chlorine dioxide is dosed, the analyser can be used to monitor the presence of chlorites both as unreacted reagent and as disinfection/oxidation by-product
- where chlorine dioxide and chlorites are removed by means of activated carbon filters, ferrous chloride or any other proper system, to verify the effectiveness of the process on chlorites removal
- at the outlet of the plant and in distribution lines, according to various contact times, to monitor actual chlorites concentration in the water intended for human consumption (even if chlorine dioxide is not the sole oxidant used in the treatment of the water).

The same analyser subject of the present patent can also be used in all those applications where chlorine dioxide or chlorites are dosed for different purposes (disinfection, oxidation, bleaching etc.)

## Claims

1. A system for continuous on-line selective analysis of chlorites in water, **characterized by**:
a) polarization voltage chosen in order to make the analyser selective for chlorites
b) electronic circuit generating the polarization voltage and receiving the signal from the sensor, not influenced by variations in process conditions
c) temperature compensation of the measure with a dedicated algorithm
d) cell to transmitter wiring not subject to interferences
e) 3 electrodes (of suitable material) potentiostatic measuring system
f) hydraulic behaviour of the sample on the electrodes able to null polarization phenomena

2. A system for continuous on-line selective analysis of chlorites in water, as claimed in claim 1. without temperature compensation

3. A system for continuous on-line selective analysis of chlorites in water, as claimed in claim 1. with high distance between electronic unit and sensor

4. A system for continuous on-line selective analysis of chlorites in water, as claimed in claim 1. with electrode holders suitable to operate with different process conditions; among them:
a) cell suitable to work in through flow arrangement with input sample flow rate up to 200 I/h and atmospheric sample pressure
b) cell suitable to work in through flow arrangement and in direct in line installation, with input sample flow higher than 60 l/h, operating pressure up to 4 bar (in line installation)

5. A system for continuous on-line selective analysis of chlorites in water, as claimed in claim 1. with Ag/AgCl reference, KCl electrolyte saturated with AgCl, or other different reference electrodes according to specific application requirements.

6. A system for continuous on-line selective analysis of chlorites in water, as claimed in claim 1. with graphite measuring electrode (W) and graphite counter electrode (C), with properly treated surfaces for ground noise reduction

7. A system for continuous on-line selective analysis of chlorites in water, as claimed in claim 1. with a polarization voltage that assures good sensitivity in measure and good signal/ground noise ratio.

8. A system for continuous on-line selective analysis of chlorites in water, as claimed in claim 7. even in presence of interfering substance typical of the possible applications

9. A system for continuous on-line selective analysis of chlorites in water, as claimed in claim 1. with sensitivity and full scale suitable for specific application needs in the typical plants where chlorites are to be measured as residual or by-products of some treatment.

10. A system for continuous on-line selective analysis of chlorites in water, as claimed in claim 1. with mechanical, physical or electronic depolarizing means.
